# EUROPÄISCHE PATENTSCHRIFT

(11) **EP 0 915 822 B1**
(45) Veröffentlichungstag und Bekanntmachung des Hinweises auf die Patenterteilung: **12.12.2001**
(21) Anmeldenummer: 97936646.5
(22) Anmeldetag: 21.07.1997
(51) Int. Cl.: C07C 59/135, C07C 51/58, B01J 19/18

(54) **VERFAHREN ZUR HERSTELLUNG VON PERFLUOR-[2-(N-PROPOXI)-PROPIONYL]FLUORID**
PROCESS FOR PREPARING PERFLUORO-[2-(N-PROPOXYL)-PROPIONYL]FLUORIDE
PROCEDE DE PREPARATION DE PERFLUORO-[2-(N-PROPOXYL)-PROPIONYLE]FLUORURE

(30) Priorität: 29.07.1996 DE 19630535; 14.08.1996 DE 19632723
(43) Veröffentlichungstag der Anmeldung: 19.05.1999
(73) Patentinhaber: Dyneon GmbH & Co. KG, 84504 Burgkirchen (DE)
(72) Erfinder: FRANZ, Raimund, D-65779 Kelkheim (DE); SIEGEMUND, Günter, D-65719 Hofheim (DE)
(86) Internationale Anmeldenummer: EP9703928
(87) Internationale Veröffentlichungsnummer: WO9804511

(56) Entgegenhaltungen:
- DE-A- 3 901 001
- US-A- 3 600 136

## Beschreibung

Die vorliegende Erfindung betrifft ein Verfahren zur kontinuierlichen, selektiven Dimerisierung von Hexafluorpropenoxid (I) zu Perfluor-[2-(n-propoxi)-propionyl]fluorid (II).

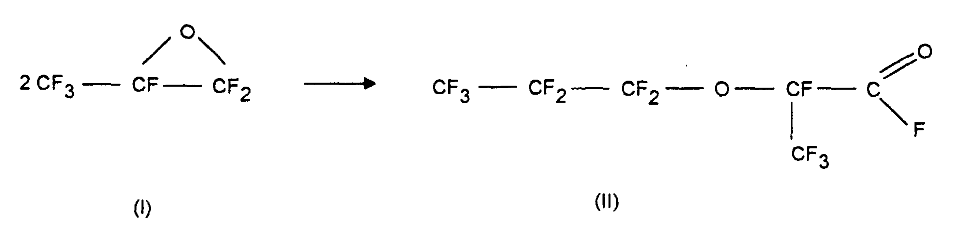

Das Ether-Carbonsäurefluorid (II) ist ein Zwischenprodukt bei der Herstellung von Perfluor-(n-propyl-vinylether), PPVE, einem wertvollen Comonomer für die Produktion von modifiziertem Polytetrafluorethylen.

Es ist bekannt, die Dimerisierung von (I) unter Verwendung von geeigneten nucleophilen Katalysatoren, wie beispielsweise Fluorionen oder tertiären Aminen, bei 0 - 30°C in dipolar-aprotischen Lösungsmitteln wie Acetonitril oder 1.2-Dimethoxiethan ablaufen zu lassen. Zuweilen werden auch Schwermetallionen wie Cu(I), Cu(II) oder Ag(I) als Cokatalysatoren eingesetzt. Alle bekannten Verfahren zur Dimerisierung von Hexafluorpropenoxid weisen jedoch Nachteile hinsichtlich der Selektivität auf. Das Reaktionsprodukt enthält zum Teil erhebliche Mengen an Perfluorpropionylfluorid; daneben beobachtet man eine partielle Weiterreaktion des an sich gewünschten Dimeren zu Tri- und Tetrameren bis hin zu perfluorierten Polyether-Carbonsäurefluoriden.

So kann zum Beispiel nach DE-A-27 56 919 die Dimerisierung von (I) zu (II) mittels einer aus Acetonitril, Acrylnitril und Kupfer(l)chlorid bestehenden Katalysatorlösung nur mit 73,7 %iger Ausbeute durchgeführt werden. Ein besseres Ergebnis ist in DE-A-39 01 001 angegeben, wonach in Gegenwart von Tetramethyl-ethylendiamin in Acetonitril bei 10 - 12°C aus Hexafluorpropenoxid ein Gemisch aus 15 Gew.-% Perfluorpropionylfluorid, 80,54 Gew.-% (II) und 4,46 Gew.-% des trimeren Perfluorether-Carbonsäurefluorids erhalten wird. Als Reaktionsapparatur wird unter anderem eine Blasensäule vorgeschlagen, in deren als Ruhezone ausgebildetem Fußteil sich das im Reaktionsmedium unlösliche, flüssige Additionsprodukt sammelt. Das darin gelöste Perfluorpropionylfluorid geht jedoch im Verlauf der weiteren Herstellung von PPVE verloren.

In JP-57-45132 wird ein Batch-Verfahren beschrieben, in dem durch die katalytische Wirkung von Fluorionen in Acetonitril oder 1,2-Dimethoxiethan durch das Vorlegen von 100 g von bereits hergestelltem Produkt (II) die Dimerisierung von weiteren 50 g (I) mit einer Selektivität bis zu 98 % abläuft. Trotz einer Nachrührzeit von 2 Stunden beträgt jedoch der maximal erreichbare Umsatz von (I) nur 89 %; daraus folgt, daß der Katalysator bereits nach einem einzigen Ansatz viel von seiner Wirkung verloren hat. Dieses Verfahren ist somit zur technischen Anwendung nicht geeignet.

Es wurde nun überraschend gefunden , daß man Perfluor-[2-(n-propoxi)propionyl]fluorid in einem kontinuierlichen Prozeß durch Dimerisierung von Hexafluorpropenoxid mit hoher Selektivität herstellen kann, wenn man die Reaktion in Anlehnung an das in DE-A-39 01 001 beschriebene Verfahren in Blasensäulen oder in mit einer Blasensäule vergleichbaren Reaktoren durchführt, jedoch das Gasgemisch, das sich dort über der Reaktionslösung befindet, mittels einer Pumpe einem externen Kreislauf unterwirft, wobei das Dimerisierungsprodukt des Hexafluorpropenoxids unmittelbar nach seiner Bildung aus dem Reaktionsgemisch ausgetrieben und extern kondensiert wird. Es wird damit einer Weiterreaktion entzogen. Hält man gleichzeitig das Kondensat in seiner Vorlage auf Rückflußtemperatur, destillieren die darin gelösten, tiefsiedenden Anteile an Hexafluorpropenoxid und/oder Perfluorpropionylfluorid ab und werden dadurch in den Gaskreislauf zurückgeführt. Man erhält auf diese Weise ein Kondensat, das ganz überwiegend aus dem gewünschten Perfluor-[2-(n-propoxi)-propionyl]-fluorid (II) besteht. Der Verbrauch von Hexafluorpropenoxid (I) kann durch Messung des Gasdruckes in der Apparatur leicht kontrolliert und durch kontinuierliches oder portionsweises Nachliefern ausgeglichen werden.

Gegenstand der vorliegenden Erfindung ist folglich ein Verfahren zur kontinuierlichen Herstellung von Perfluor-[2-(n-propoxi)-propionyl]fluorid (PPPF) durch Dimerisierung von Hexafluorpropenoxid in einer Blasensäule oder einem damit vergleichbaren, mit einem dipolar-aprotischen Lösungsmittel sowie einem nucleophilen Katalysator gefüllten Reaktor, dadurch gekennzeichnet, daß man
a) am Boden des Reaktors Hexafluorpropenoxid einleitet,
b) das im Reaktor befindliche Gasgemisch mittels einer Pumpe im Kreis führt und dadurch das PPPF vor einer Weiterreaktion austreibt,
c) das am Kopf des Reaktors austretende Gasgemisch in einem Kondensator abkühlt,
d) die in Schritt c) nicht kondensierten Bestandteile des Gasgemischs wieder dem Reaktor zuführt,
e) das in Schritt c) erhaltene Kondensat, das aus einer oberen Lösungsmittelphase und einer unteren PPPF-Phase besteht, zwecks Beseitigung tiefsiedender Bestandteile auf Rückflußtemperatur hält,
f) das verbrauchte Hexafluorpropenoxid gemeinsam mit den nicht kondensierten Bestandteilen des Gasgemischs gemäß Schritt d) in den Gaskreislauf nachliefert, und
g) aus dem Kondensat nach Schritt e) die PPPF-Phase als Produkt entnimmt und die Lösungsmittelphase wieder dem Reaktor zuführt.

Als Reaktor für das erfindungsgemäße Verfahren ist jeder temperierbare Flüssigkeits-Behälter verwendbar, in welchem ein Gas eingeleitet, dort fein verteilt und aus der Flüssigkeits-Oberfläche wieder ausgetrieben werden kann. Da der Prozeß keine außergewöhnlichen Anforderungen hinsichtlich Druck und Temperatur stellt, ist für den Reaktor, die Gasleitungen, die Kühler und die Vorlage jedes Material zulässig, das gegenüber dem Produkt und den zu verwendenden Katalysatoren chemisch beständig ist. Es kommen folglich rostfreier Stahl, Nickel und seine Legierungen, Edelmetalle, jedoch auch Polyolefin-Kunststoffe sowie Borsilikatglas in Frage. Bevorzugt werden rostfreier Stahl und Polyolefin-Kunststoffe eingesetzt. Um die Reaktionstemperatur konstant zu halten, ist es vorteilhaft, den Reaktor mit einer Ummantelung oder mit einem eintauchenden Wärmetauscher für Heiz- bzw. Kühlflüssigkeit zu versehen. Die Gasverteilung im Reaktor kann nach dem Stand der Technik mittels eines geeigneten Rührwerks, mittels Düsen oder durch Verwendung von Gaseinleitungs-Fritten aus Sintermaterial erfolgen. Mit Vorteil werden daher für den Prozeß Rührkessel mit Begasungsrührer oder Blasensäulen eingesetzt.

Der Reaktor wird in Anlehnung an den Stand der Technik mit einem dipolar-aprotischen Lösungsmittel sowie einem Katalysator befüllt. Der Siedepunkt des Lösungsmittels liegt zwischen 50 und 160 °C. Es ist für das erfindungsgemäße Verfahren vorteilhaft, wenn der Siedepunkt des Lösungsmittels im Bereich von 50 - 100°C, bevorzugt im Bereich von 60 - 90°C liegt. Bevorzugte Lösungsmittel sind Carbonsäurenitrile mit 2 bis 6 C-Atomen wie Acetonitril und Propionitril, sowie 1,2-Dimethoxiethan, insbesondere jedoch Acetonitril. Alle in der oben angeführten Literatur genannten Katalysatoren, wie Alkalimetallfluoride und/oder tertiäre Amine, sind bei der erfindungsgemäßen Verfahrensweise einzeln oder in Kombination verwendbar. Ihre Konzentration bezogen auf das Lösungsmittel liegt im Bereich von 0,1 - 10 Gew.-%, bevorzugt im Bereich von 0,2 - 2,0 Gew.-%.

Als Reaktionstemperatur eignet sich bei der erfindungsgemäßen Verfahrensweise der Bereich von 0 - 60°C; bevorzugt ist jedoch der Bereich von 10 - 40°C, insbesondere 15 - 25°C.

Zur Erzeugung des Gaskreislaufes eignen sich alle dem Stand der Technik entsprechenden Gaspumpentypen, deren produktberührte Teile gegenüber dem umgepumpten Reaktionsgemisch, das aus Säurefluoriden sowie Hexafluorpropenoxid besteht, chemisch beständig sind. Die Menge des pro Zeiteinheit umgepumpten Gases richtet sich nach dem Volumen der Lösung im Reaktor und nach deren Temperatur; sie muß so eingestellt werden, daß sich das Produkt, im Gegensatz zum Stand der Technik, nicht als schwere Phase am Boden des Reaktors sammeln kann.

Der Druck des im Kreis geführten Gasgemisches liegt bei der erfindungsgemäßen Verfahrensweise vorteilhaft in einem Bereich von 100 mbar unter und über dem Atmosphärendruck, bevorzugt ist ein Überdruck von 5 - 50 mbar. Er wird zweckmäßig in der Gasatmosphäre über der Flüssigkeitsoberfläche des Reaktionsgemischs gemessen. Über die Messung dieses Druckes wird das Nachliefern des verbrauchten Hexafluorpropenoxids geregelt.

Auch die Abtrennung des dimeren Reaktionsproduktes aus dem im Kreis geführten Gasgemisch durch Kondensation erfolgt gemäß dem Stand der Technik in Abhängigkeit vom Produkt-Durchsatz und von der Gas-Umlaufgeschwindigkeit. Es ist bekannt, daß nach Vorgabe der genannten Parameter sowie der Kühlmitteltemperatur die erforderliche Kühlfläche berechnet werden kann.

Bei der erfindungsgemäßen Verfahrensweise verläßt das im Kühler erzeugte Kondensat den im Kreis geführten Gasstrom, durchläuft einen Rückflußkühler und sammelt sich in einer Vorlage. Diese Vorlage wird beheizt, so daß ihr Inhalt am Rückfluß siedet und gelöste, tiefsiedende Anteile abdestillieren und durch den Rückflußkühler in das im Kreis geführte Gasgemisch zurückgelangen. Da das Kondensat jedoch stets auch Anteile des im Reaktor eingesetzten, mit dem Produkt nicht mischbaren Lösungsmittels mit sich führt, weist die Vorlage für das Kondensat zweckmäßig auch eine Möglichkeit zur kontinuierlichen Phasentrennung auf. Die leichtere Lösungsmittel-Phase kann mittels einer Pumpe abgesaugt und kontinuierlich in den Reaktor zurückgegeben werden, während die schwere Produktphase ebenso kontinuierlich abgelassen werden kann.

Das folgende Ausführungsbeispiel soll die erfindungsgemäße Verfahrensweise erläutern.

### Beispiel

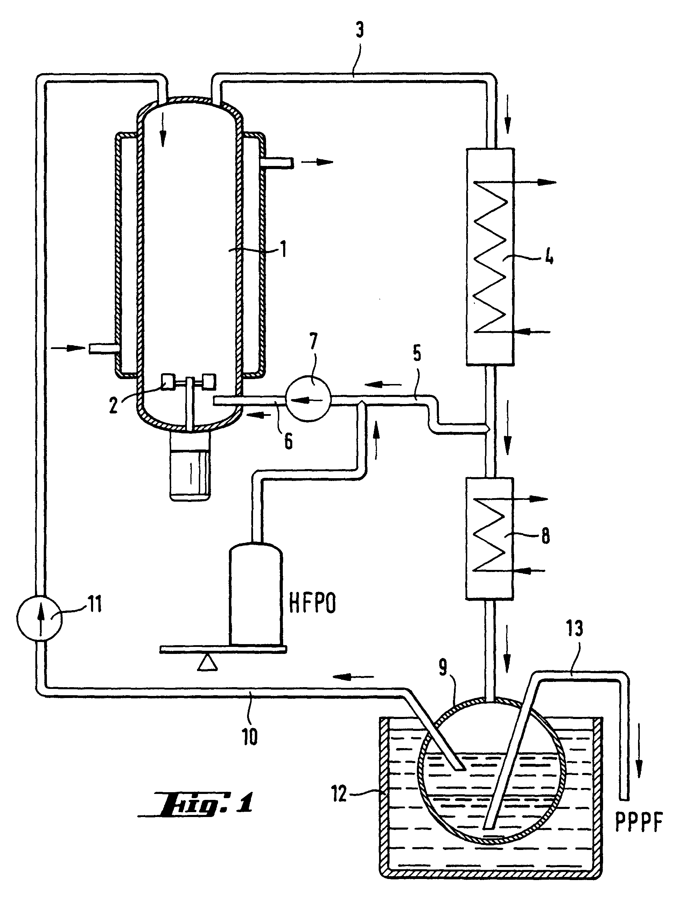

Eine Apparatur wurde gemäß dem in Fig. 1 dargestellten Schema erstellt. Der ummantelte Rohrreaktor (1) aus rostfreiem Stahl (L = 80 cm, LW = 8 cm) wies zwei Schaugläser sowie am Boden ein schneilaufendes Rührwerk (2) zur Gasverteilung auf. In diesen Reaktor (1) wurde eine Lösung von 25 g **N,N,N',N'**-Tetramethylethylendiamin in 2,5 kg Acetonitril eingefüllt und auf 20°C temperiert. Der Kopf des Reaktors (1) war über die Gas-Rohrleitung (3) in dem in Fig. 1 gezeigten Sinne mit dem Kondensator (4) (Kühlmitteltemperatur: -10°C), und dieser über die Leitungen (5) und (6) mit der Gaspumpe (7) und dem Boden des Reaktors (1) verbunden, so daß ein Gaskreislauf zustande kam. Aus einer auf einer Waage stehenden Vorrats-Druckflasche wurde Hexafluorpropenoxid HFPO an der in Fig. 1 bezeichneten Stelle in den Gaskreislauf eingeleitet und mittels des Rührwerks im Reaktor (1) verteilt. Die im Reaktor (1) gebildeten höhersiedenden Gasanteile wurden im Kondensator (4) verflüssigt, passierten den Rückflußkühler (8) (Kühlmitteltemperatur: 0°C) und gelangten in die Vorlage (9), in welcher sie sich in 2 flüssige Phasen trennten. Die leichtere Phase bestand überwiegend aus Acetonitril und wurde über die Rohrleitung (10) mittels der Flüssigkeitspumpe (11) in den Reaktor (1) zurückgepumpt. Der Inhalt der Vorlage (9) wurde mit dem Heizbad (12) (Badtemperatur: 80°C) am Rückfluß gehalten, um tiefsiedende, gelöste Anteile auszutreiben. Von Zeit zu Zeit wurde mit Hilfe des bis zum Boden der Vorlage (9) eintauchenden Rohres (13) die schwerere der beiden Flüssigphasen abgesaugt und gesammelt. Die Zusammensetzung dieses Materials wurde in regelmäßigen Abständen mittels ¹⁹F-NMR analysiert. Es bestand zu 97 - 98 Mol.-% aus Perfluor-[2-(n-propoxi)-propionyl]fluorid; der Gehalt an Perfluorpropionylfluorid überstieg den Wert von 1 Mol-% nicht.

## Patentansprüche

1. Verfahren zur kontinuierlichen Herstellung von Perfluor-[2-(n-propoxi)propionyl]fluorid (PPPF) durch Dimerisierung von Hexafluorpropenoxid in einer Blasensäule oder einem damit vergleichbaren, mit einem dipolar-aprotischen Lösungsmittel sowie einem nucleophilen Katalysator gefüllten Reaktor, **dadurch gekennzeichnet, daß** man
a) am Boden des Reaktors Hexafluorpropenoxid einleitet,
b) das im Reaktor befindliche Gasgemisch mittels einer Pumpe im Kreis führt und dadurch das PPPF vor einer Weiterreaktion austreibt,
c) das am Kopf des Reaktors austretende Gasgemisch in einem Kondensator abkühlt,
d) die in Schritt c) nicht kondensierten Bestandteile des Gasgemischs wieder dem Reaktor zuführt,
e) das in Schritt c) erhaltene Kondensat, das aus einer oberen Lösungsmittelphase und einer unteren PPPF-Phase besteht, zwecks Beseitigung tiefsiedender Bestandteile auf Rückflußtemperatur hält,
f) das verbrauchte Hexafluorpropenoxid gemeinsam mit den nicht kondensierten Bestandteilen des Gasgemischs gemäß Schritt d) in den Gaskreislauf nachliefert, und
g) aus dem Kondensat nach Schritt e) die PPPF-Phase als Produkt entnimmt und die Lösungsmittelphase wieder dem Reaktor zuführt.

2. Verfahren nach Anspruch 1, **dadurch gekennzeichnet, daß** der Reaktor mit einer Vorrichtung zur Konstanthaltung der Temperatur ausgestattet ist.

3. Verfahren nach einem der Ansprüche 1 oder 2, **dadurch gekennzeichnet, daß** die Reaktionstemperatur zwischen 0 und 60°C liegt.

4. Verfahren nach einem der Ansprüche 1 bis 3, **dadurch gekennzeichnet, daß** die Reaktionstemperatur zwischen 10 und 40°C liegt.

5. Verfahren nach einem der Ansprüche 1 bis 4, **dadurch gekennzeichnet, daß** die Reaktionstemperatur zwischen 15 und 25°C liegt.

6. Verfahren nach einem der Ansprüche 1 bis 5, **dadurch gekennzeichnet, daß** der Siedepunkt des dipolar-aprotischen Lösungsmittels zwischen 50 und 100°C liegt.

7. Verfahren nach einem der Ansprüche 1 bis 6, **dadurch gekennzeichnet, daß** der Siedepunkt des dipolar-aprotischen Lösungsmittels zwischen 60 und 90°C liegt.

8. Verfahren nach einem der Ansprüche 1 bis 7, **dadurch gekennzeichnet, daß** das dipolar-aprotische Lösungsmittel ein Carbonsäurenitril mit 2 bis 6 C-Atomen ist.

9. Verfahren nach einem der Ansprüche 1 bis 8, **dadurch gekennzeichnet, daß** das dipolar-aprotische Lösungsmittel Acetonitril, Propionitril oder 1,2-Dimethoxiethan ist.

10. Verfahren nach einem der Ansprüche 1 bis 9, **dadurch gekennzeichnet, daß** der nucleophile Katalysator in einer Menge von 0,1 bis 10 Gew.-% bezogen auf das Lösungsmittel vorhanden ist.

11. Verfahren nach einem der Ansprüche 1 bis 10, **dadurch gekennzeichnet, daß** der nucleophile Katalysator in einer Menge von 0,5 bis 2,0 Gew.-% bezogen auf das Lösungsmittel vorhanden ist.

12. Verfahren nach einem der Ansprüche 1 bis 11, **dadurch gekennzeichnet, daß** der Druck des im Kreis geführten Gasgemisches in einem Bereich von 100 mbar oberhalb und unterhalb des Atmosphärendrucks liegt.

13. Verfahren nach einem der Ansprüche 1 bis 12, **dadurch gekennzeichnet, daß** der Druck des im Kreis geführten Gasgemisches in einem Bereich von 5 bis 50 mbar oberhalb des Atmosphärendrucks liegt.

14. Verfahren nach einem der Ansprüche 1 bis 13, **dadurch gekennzeichnet, daß** die Vorlage für das Kondensat eine Vorrichtung zur kontinuierlichen Phasentrennung aufweist.

## Claims

1. A process for the continuous preparation of perfluoro[2-(n-propoxy)propionyl] fluoride (PPPF) by dimerizing hexafluoropropene oxide in a bubble column or a comparable reactor charged with a dipolar aprotic solvent and a nucleophilic catalyst, which comprises
a) introducing hexafluoropropene oxide at the bottom of the reactor,
b) circulating the gas mixture present in the reactor by means of a pump and thereby driving out the PPPF before further reaction occurs,
c) cooling the gas mixture leaving the top of the reactor in a condenser,
d) returning the constituents of the gas mixture which are not condensed in step c) to the reactor,
e) maintaining the condensate obtained in step c), which consists of an upper solvent phase and a lower PPPF phase, at reflux temperature to eliminate low-boiling constituents,
f) feeding the amount of hexafluoropropene oxide consumed together with the uncondensed constituents of the gas mixture as described in step d) into the gas circuit and
g) taking the PPPF phase as product from the condensate after step e) and returning the solvent phase to the reactor.

2. The process as claimed in claim 1, wherein the reactor is equipped with a facility for keeping the temperature constant.

3. The process as claimed in claim 1 or 2, wherein the reaction temperature is from 0 to 60°C.

4. The process as claimed in any one of claims 1 to 3, wherein the reaction temperature is from 10 to 40°C.

5. The process as claimed in any one of claims 1 to 4, wherein the reaction temperature is from 15 to 25°C.

6. The process as claimed in any one of claims 1 to 5, wherein the boiling point of the dipolar aprotic solvent is from 50 to 100°C.

7. The process as claimed in any one of claims 1 to 6, wherein the boiling point of the dipolar aprotic solvent is from 60 to 90°C.

8. The process as claimed in any one of claims 1 to 7, wherein the dipolar aprotic solvent is a carboxylic acid nitrile having from 2 to 6 carbon atoms.

9. The process as claimed in any one of claims 1 to 8, wherein the dipolar aprotic solvent is acetonitrile, propionitrile or 1,2-dimethoxyethane.

10. The process as claimed in any one of claims 1 to 9, wherein the nucleophilic catalyst is present in an amount of from 0.1 to 10% by weight, based on the solvent.

11. The process as claimed in any one of claims 1 to 10, wherein the nucleophilic catalyst is present in an amount of from 0.5 to 2.0% by weight, based on the solvent.

12. The process as claimed in any one of claims 1 to 11, wherein the pressure of the circulated gas mixture is in a range from 100 mbar above to 100 mbar below atmospheric pressure.

13. The process as claimed in any one of claims 1 to 12, wherein the pressure of the circulated gas mixture is in a range of from 5 to 50 mbar above atmospheric pressure.

14. The process as claimed in any one of claims 1 to 13, wherein the receiver for the condensate has a facility for continuous phase separation.

## Revendications

1. Procédé pour la préparation en continu de perfluoro-[2-(n-propoxy)-propionyl]fluorure (PPPF) par dimérisation d'oxyde d'hexafluoropropène dans une colonne à bulles ou dans un réacteur comparable rempli avec un solvant dipolaire aprotique et un catalyseur nucléophile, **caractérisé en ce qu'**on
a) introduit l'oxyde d'hexafluoropropène à la base du réacteur,
b) amène le mélange gazeux se trouvant dans le réacteur dans la boucle au moyen d'une pompe et **en ce qu'**on expulse le PPPF avant une réaction ultérieure,
c) refroidit le mélange de gaz débordant en tête du réacteur dans un condensateur,
d) introduit de nouveau dans le réacteur les constituants du mélange gazeux non condensés à l'étape c),
e) maintient le condensat obtenu à l'étape c), qui est constitué d'une phase solvant supérieure et d'une phase PPPF inférieure, à la température de reflux pour éliminer les constituants à bas point de fusion,
f) réintroduit l'oxyde d'hexafluoropropène utilisé en même temps que les constituants non condensés du mélange gazeux de l'étape d) dans le circuit des gaz, et
g) on prélève du condensat selon l'étape e) la phase PPPF comme produit et on réintroduit la phase solvant dans le réacteur.

2. Procédé selon la revendication 1, **caractérisé en ce que** le réacteur est équipé avec un dispositif pour maintenir la température constante.

3. Procédé selon l'une des revendications 1 ou 2, **caractérisé en ce que** la température de réaction se situe entre 0 et 60°C.

4. Procédé selon l'une des revendications 1 à 3, **caractérisé en ce que** la température de réaction se situe entre 10 et 40°C.

5. Procédé selon l'une des revendications 1 à 4, **caractérisé en ce que** la température de réaction se situe entre 15 et 25°C.

6. Procédé selon l'une des revendications 1 à 5, **caractérisé en ce que** le point d'ébullition du solvant dipolaire aprotique se situe entre 50 et 100°C.

7. Procédé selon l'une des revendications 1 à 6, **caractérisé en ce que** le point d'ébullition du solvant dipolaire aprotique se situe entre 60 et 90°C.

8. Procédé selon l'une des revendications 1 à 7, **caractérisé en ce que** le solvant dipolaire aprotique est un nitrile d'acide carboxylique ayant de 2 à 6 atomes de carbone.

9. Procédé selon l'une des revendications 1 à 8, **caractérisé en ce que** le solvant dipolaire aprotique est l'acétonitrile, le propionitrile ou le 1,2-diméthoxyéthane.

10. Procédé selon l'une des revendications 1 à 9, **caractérisé en ce que** le catalyseur nucléophile est présent en une quantité de 0,1 à 10 % en poids par rapport au solvant.

11. Procédé selon l'une des revendications 1 à 10, **caractérisé en ce que** le catalyseur nucléophile est présent en une quantité de 0,5 à 2,0 % en poids par rapport au solvant.

12. Procédé selon l'une des revendications 1 à 11, **caractérisé en ce que** la pression du mélange gazeux introduit dans la boucle se situe dans une gamme comprise entre 100 mbar au-dessus et au-dessous de la pression atmosphérique.

13. Procédé selon l'une des revendications 1 à 12, **caractérisé en ce que** la pression du mélange gazeux introduit dans la boucle se situe dans une gamme comprise entre 5 et 50 mbar au-dessus de la pression atmosphérique.

14. Procédé selon l'une des revendications 1 à 13, **caractérisé en ce que** le récepteur du condensat présente un dispositif pour la séparation de phases en continu.
